# EUROPEAN PATENT APPLICATION

(11) **EP 3 622 887 A1**
(43) Date of publication of application: **18.03.2020**
(21) Application number: 19206164.6
(22) Date of filing: 28.10.2015
(51) Int. Cl.: A61B 5/00, A61C 1/00, B29C 67/00

(54) **MODELLING SYSTEM**

(30) Priority: 03.11.2014 EP 14191469
(62) Divisional of application: 15787195.5
(71) Applicant: Unilever PLC, London Greater London EC4Y 0DY (GB); Unilever N.V., 3013 AL Rotterdam (NL)
(72) Inventor: ASHCROFT, Alexander Thomas, Cheshire, CH1 6LD (GB); CARAO, Jorge, Luis, Wirral, CH62 2EL (GB); HAYES, Rebecca, Anne, Bebington, Wirral, Merseyside CH63 3JW (GB); PERUFFO, Massimo, Swindon, SN4 8FQ (GB)
(74) Representative: Tansley, Sally Elizabeth

(57) **Abstract**

A product for demonstrating the impact of a treatment on a surface, the product prepared by the steps of:
i optionally imaging at least one untreated surface
ii applying at least one treatment to the surface(s)such that if step i) is not performed at least two different surfaces are treated with differing treatments,
iii imaging the treated surface(s) to create an image;
iv converting the imaging data into a format suitable to create a magnified image on a 3D printer;
v producing a 3D model of each of the imaged surface(s).

## Description

The present invention relates to a method of demonstrating the impact of a treatment on a surface.

WO14041186A1 describes a system or component such as software for 3D modelling of bodies.

Consumers are aware of the damage that aging and particular aggressive beauty treatments can cause to the surface of their bodies or possessions. Although, especially in personal care, remedial treatments are available to mitigate these detrimental effects, the concept can be difficult for the consumer to grasp and the full effects of the remedial treatment difficult to fully comprehend.

The present invention relates to a method of demonstrating the impact of a treatment on a surface comprising the steps of:
i. optionally imaging at least one untreated surface,
ii. applying at least one treatment to the surface(s), such that if step i) is not performed at least two different surfaces are treated with differing treatments,
iii. imaging the surface(s) to create image(s),
iv. converting the imaging data into a format suitable to produce a magnified image on a 3D printer,
v. producing magnified 3D models of the imaged surfaces.

In the context of the present invention the term untreated surface means a surface that has not been treated with a product that damages the surface within two hours.

In one embodiment of the invention the image is a topographic surface image; preferably, the topographic surface image is produced using a laser profilometer.

The topographic surface is converted into a format suitable for a 3D printer, preferably it is exported in a digital file as spatial coordinates (X, Y, Z) of each point which describes the topographic (3D) surface. Preferably, this is completed using a profilometer. An example of a suitable profilometer is the Sensofar S neox profilometer that can produce a 3d image of the surface to be studied. The profilometer software, for example sensoSCAN v5, can export a file ".dat" that is a list of all the X, Y, Z coordinates of each point.

Preferably the conversion of the imaging data iv) comprises a magnification process. The magnification is preferably achieved by change of resolution, units and/or rescale of coordinate axis, producing a new digital file with the new spatial coordinates. A preferred way of magnifying the data points is using Matlab. In this preferred method the ".dat" file is imported in Matlab as a matrix and a set of Matlab scripts are used to manipulate the matrix and change the resolution/scale. It is highly preferred if the matrix is exported into a new ASCII file ".XYZ" as a list of all the X, Y, Z coordinates of each point.

The imaging data is converted into an image suitable for a 3D printer. Preferably, the file is imported in a 3D-CAD software and the 3D surface is applied onto a face of a parallelogram to obtain a 3D object. The resulting 3D image is exported to a digital file compatible with the 3D-printer device software. The XYZ file is preferably imported into the "Rhino" software package, which can convert it into a 3d file and export as a .STL file.

The 3D image is printed to form a 3d object. This can be achieved by using an EOS (Electro Optical Systems) EOSINT P380 Selective Laser Sintering printer and a 3D replica of the magnified surface produced.

Preferably, the surface that is to be imaged is part of a human body, more preferably the skin or the teeth.

Most preferably the surface is tooth enamel. This is due to the difficulty the consumer has in perceiving the erosion of enamel and the beneficial effect certain treatments can have. This method is particularly beneficial in demonstrating the formation of hydroxyapatite on the tooth surface. Suitable hydroxyapatite generation methods are described in WO2008/068149, WO2011/110414 and WO2011/160996.

In one aspect of the invention an untreated surface is compared with a treated surface.

In a second aspect of the invention surfaces treated with two different treatment products are compared.

In a further embodiment of the invention more than one treatment is applied to the surface, preferably the enamel. This can be part of a two-step process to treat the surface or can be to show differing effects of two alternative treatments so can be different treatments are applied to different areas or samples of enamel.

The latter method could be used to show the effects of brushing tooth minerals, such as enamel or dentine, with toothpastes of differing abrasivity.

The invention will now be illustrated by the following non-limiting Examples:

### Examples

The roots of human extracted incisors and premolars were removed by using a diamond abrasion wheel. The facial surface was then flattened using a high abrasivity disc (Tycet Ltd, Hemel Hempstead, Herts, UK) until it was flat enough to fit a block of enamel and dentine split approximately 50:50, of size 4 x 4mm. The lingual part of the tooth was then cut with a Two Well Model 3242 Wire Cutter (Ebner, Le Locle, Switzerland) to leave a slice approximately 2.5mm thick. The specimen was prepared planar parallel. The surface was then polished sequentially with 3 and 1 µm diamond suspensions (Kemet International Ltd, Maidstone, Kent, UK) and given a final polish with 0.3 µm Micropolish II (Buehler, Coventry, UK). Specimens were then sonicated for 5 mins, rinsed with Milli Q water (Millipore, UK) and then sonicated again for 5 mins to remove any smear layer formed during the polishing process.

The untreated specimens were imaged according to the method below.

The specimens were mounted in a modified Martindale brushing machine (Goodbrand-Jeffreys Ltd., Stockport, UK) fitted with flat-trimmed benefit toothbrushes. In this apparatus the brush heads describe Lissajous' figures, which combine linear and elliptical motions to ensure comprehensive coverage of the brushed specimen. The enamel specimen from the image was brushed with a slurry of a toothpaste of known abrasivity. The toothpaste was pre-mixed with water and 0.5% w/w sodium carboxymethyl cellulose (SCMC) solution in the ratio 1:1:1 paste:water:SCMC. Toothpaste slurry (10ml) was added to the well of the brushing machine and the enamel-dentine specimens brushed for 2 x ten minutes at 150 cycles/min at an applied load of 275g. The image for the model was taken from the enamel side of the specimen.

### Imaging Method

The topographic surface was converted into a format suitable for a 3D printer, by exporting in a digital file as spatial coordinates (X, Y, Z) of each point which describes the topographic (3D) surface using a sensoSCAN v5 with a Sensofar S neox profilometer.

The resulting digital file data was magnified by importing to Matlab as a matrix and using Matlab scripts to manipulate the matrix and change the resolution/scale. The matrix is then exported into a new ASCII file ".XYZ" as a list of all the X,Y,Z coordinates of each point.

The imaging data is converted into an image suitable for a 3D printer using 3D-CAD software and the 3D surface is applied onto a face of a parallelogram to obtain a 3D object. The resulting 3D image is exported to a digital file compatible with the 3D-printer device software by use of the "Rhino" software package, which can convert it into a 3d file and export as a .STL file.

The 3D image is printed to form a 3d object. This is achieved by using an EOS (Electro Optical Systems) EOSINT P380 Selective Laser Sintering printer and a 3D replica of the magnified surface produced.

Comparing the two 3D images demonstrated the effect of the toothpaste on the tooth enamel.

## Claims

1. A product for demonstrating the impact of a treatment on a surface, the product prepared by steps comprising:
i optionally imaging at least one untreated surface,
ii applying at least one treatment to the surface(s), such that if step i) is not performed at least two different surfaces are treated with differing treatments,
iii. imaging the treated surface(s) to create an image,
iv. converting the imaging data into a format suitable to create a magnified image for a 3D printer,
v. producing a magnified 3D model of the imaged surface(s).

2. A product according to claim 1 in which the image iii) is a topographical image.

3. A product according to any preceding claim in which an untreated surface is compared with a treated surface.

4. A product according to any preceding claim in which surfaces treated with two different treatment products are compared.

5. A product according to any preceding claim in which the surface is part of a human body.

6. A product according to any preceding claim in which the surface is tooth enamel.

7. A product according to claim 6 in which the treatment is an enamel regeneration treatment.

8. A product according to claim 7 in which the treatment demonstrated is formation of hydroxyapatite on a tooth surface.

9. A product according to any preceding claim in which more than on treatment is applied to the enamel.

10. A product according to claim 9 in which different treatments are applied to different areas or samples of enamel.

11. A product according to claim 10 in which surfaces are brushed by toothpastes of differing abrasivity.
